(19) Europäisches Patentamt
European Patent Office
Office européen des brevets

(11) Veröffentlichungsnummer: **0 054 149**
**B1**

(12) # EUROPÄISCHE PATENTSCHRIFT

(45) Veröffentlichungstag der Patentschrift: **16.05.84**

(51) Int. Cl.³: **C 07 F 9/18**, A 01 N 57/14

(21) Anmeldenummer: **81108809.5**

(22) Anmeldetag: **23.10.81**

(54) **Trifluormethoxyphenyl-(di)thiophosphorsäureester, Verfahren zu ihrer Herstellung und ihre Verwendung zur Bekämpfung von Schädlingen.**

(30) Priorität: **16.12.80 DE 3047242**

(43) Veröffentlichungstag der Anmeldung: **23.06.82 Patentblatt 82/25**

(45) Bekanntmachung des Hinweises auf die Patenterteilung: **16.05.84 Patentblatt 84/20**

(84) Benannte Vertragsstaaten: **AT BE CH DE FR GB IT LI LU NL SE**

(56) Entgegenhaltungen:
**FR - A - 2 354 336**
**US - A - 3 755 511**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Seufert, Walter, Dr., Bruesseler Ring 28, D-6700 Ludwigshafen (DE)**
Erfinder: **Kiehs, Karl, Dr., Sudetenstrasse 22, D-6840 Lampertheim (DE)**
Erfinder: **Husslein, Gerd, Dr., Herrenbergstrasse 2, D-6702 Bad Duerkheim (DE)**
Erfinder: **Varwig, Juergen, Dr., Am Goetzenberg 1, D-6900 Heidelberg (DE)**
Erfinder: **Hamprecht, Gerhard, Dr., Rote-Turm-Strasse 28, D-6940 Weinheim (DE)**
Erfinder: **Adolphi, Heinrich, Dr., Kalmitweg 11, D-6703 Limburgerhof (DE)**

**EP 0 054 149 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Trifluormethoxyphenyl-(di)thiophosphorsäureester, Verfahren zu ihrer Herstellung und ihre Verwendung zur Bekämpfung von Schädlingen

Die Erfindung betrifft Trifluormethoxyphenyl-(di)thiophosporsäureester, einVerfahren zu ihrer Herstellung und Schädlingsbekämpfungsmittel, die diese Verbindungen als Wirkstoffe enthalten.

Es ist bekannt, daß O,O-Dialkyl-O-phenyl-phosphorthioate insektizid und akarizid wirksam sind (DE-PS 814 152). Außerdem ist bekannt, daß O,O-Dialkyl-O(S)-(halogenethyl-phenyl)-(di)(thio)phosphate insektizid wirksam sind (US-PS 3 755 511). Weiterhin ist bekannt, daß 4-Trifluormethylmercapto-phenyl-(di)thiophosphorsäureester insektizide und akarizide Wirkung zeigen (FR-A-2 354 336).

Es wurde gefunden, daß Trifluormethoxyphenyl-(di)thiophosporsäureester der Formel

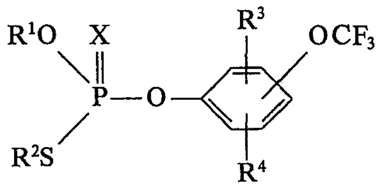

(I)

in der

| | |
|---|---|
| $R^1$ | Alkyl mit 1 bis 3 Kohlenstoffatomen, |
| $R^2$ | Alkyl oder Halogenalkyl mit 1 bis 5 Kohlenstoffatomen, Alkoxyalkyl oder Alkylthioalkyl mit 2 bis 6 Kohlenstoffatomen oder Cycloalkyl mit 3 bis 6 Kohlenstoffatomen, |
| $R^3$ und $R^4$ | unabhängig voneinander Wasserstoff, Halogen, Nitro, Cyano, Alkyl, Alkoxy oder Alkylthio mit 1 bis 4 Kohlenstoffatomen und |
| X | Sauerstoff oder Schwefel bedeuten, |

Schädlinge aus der Klasse der Insekten, Spinnentiere und Nematoden wirksam bekämpfen. Sie sind den bekannten O,O-Dialkyl-O-phenyl-thiophosphorsäureestern in ihrer Wirkung überlegen.

In Formel I stehen die Substituenten $R^1$ für Alkylreste mit 1 bis 3 Kohlenstoffatomen, wie Methyl, Ethyl, n-Propyl, i-Propyl, $R^2$ für Alkyl- oder Halogenalkylrest mit 1 bis 5 Kohlenstoffatomen, wie Methyl, Ethyl, n-Propyl, i-Propyl, n-Butyl, sec-Butyl, i-Butyl, n-Pentyl, l-Methyl-n-butyl, 2-Chlorethyl, 3-Chlorn-propyl, für Alkoxyalkyl- oder Alkylthioalkylreste mit 2 bis 6 Kohlenstoffatomen, wie 2-Methoxyethyl, 2-Ethoxyethyl, 2-Isopropoxyethyl, 2-Methylthioethyl, 2-Ethylthioethyl, oder für Cycloalkylreste mit 3 bis 6 Kohlenstoffatomen, wie Cyclopentyl, Cyclohexyl, sowie $R^3$ und $R^4$, die verschieden sein können, für Wasserstoff, Halogen, wie Chlor, Brom oder Fluor, für Nitro oder Cyano oder für Alkyl, Alkoxy oder Alkylthio mit 1 bis 4 Kohlenstoffatomen, vorzugsweise mit 1 und 2 Kohlenstoffatomen, wie Methyl oder Ethyl.

Bevorzugte Verbindungen der Formel I sind solche, bei denen $R^1$ Ethyl, $R^2$ Alkyl mit 3 oder 4 Kohlenstoffatomen, wie n-Propyl, i-Propyl, sec.-Butyl, i-Butyl, $R^3$ und $R^4$ unabhängig voneinander Wasserstoff oder Chlor und X Sauerstoff oder Schwefel bedeuten.

Man erhält die Trifluormethoxyphenyl-(di)thiophosphorsäureester der Formel I durch Umsetzung von O,S-Dialkylphosphorsäureesterchloriden der Formel

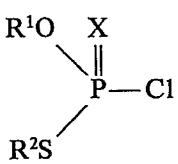

(II)

in der $R^1$, $R^2$ und X die oben angegebenen Bedeutungen haben,
gegebenenfalls in Gegenwart eines säurebindenden Mittels und in Gegenwart eines Verdünnungsmittels mit einem Trifluormethoxyphenol der Formel

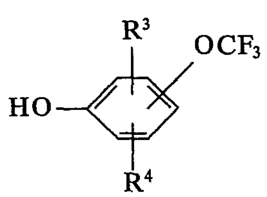

(III)

in der $R^3$ und $R^4$ die obengenannten Bedeutungen haben,

oder gegebenenfalls in Gegenwart eines Verdünnungsmittels mit einem Salz eines Trifluormethoxyphenols der Fornmel III.

Der Reaktionsablauf kann durch das folgende Formelschema wiedergegeben werden:

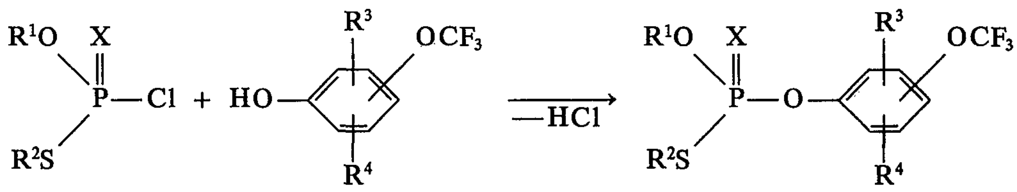


Die Umsetzung von Phosphorsäureesterchlorid der Formel II mit Phenol der Formel III kann in einem organischen Verdünnungsmittel, wie Acetonitril, Toluol, Methylethylketon, oder in zwei-Phasen-Systemen, wie Toluol/Wasser, Dichlormethan/Wasser, durchgeführt werden.

Zweckmäßigerweise setzt man die 1- bis 2-molare Menge eines säurebindenden Mittels, bezogen auf Phenol der Formnel III, zu. Vorzugsweise wird ein Überschuß von etwa 10% verwendet. Geeignet sind Basen, wie Alkalimetallcarbonate, z. B. Kaliumcarbonat, Alkalihydroxide, z. B. Natriumhydroxid, oder tertiäre Amine, z. B. Triethylamin. Anstelle von Base und Phenol kann ebenso ein Salz des Phenols mit den Phosphorsäureesterchlorid umgesetzt werden. Als Salze kommen Alkalimetallsalze, Erdalkalimetallsalze oder gegebenenfalls substituierte Ammoniumsalze, beispielsweise durch Alkyl substituierte Ammoniumsalze, wie das Natrium-, Kalium-, Calcium-, Ammonium-oder Trimethylammoniumsalz, in Betracht.

Die Reaktionstemperatur kann in einem größeren Bereich variiert werden. Im allgemeinen arbeitet man zwischen 0 und 100° C, vorzugsweise zwischen 20 und 70° C. Man läßt die Umsetzung im allgemeinen unter Normaldruck ablaufen.

Zur Durchführung des Verfahrens setzt man die Ausgangsstoffe in äquimolarem Verhältnis ein. Ein Überschuß der einen oder anderen Reaktionskomponente kann in einigen Fällen Vorteile bringen. Vorzugsweise verwendet man auf 1 Mol Phenol 0,9 bis 1,1 Mole Phosphorsäureesterchlorid.

Das Reaktionsgemisch wird in üblicher Weise aufgearbeitet, z. B. durch Versetzen mit Wasser und Trennen der Phasen. Die Rohprodukte können durch Destillation oder Säulenchromatographie gereinigt werden.

O,S-Dialkylphosphorsäureesterchloride sind bekannt und können nach bekannten Verfahren hergestellt werden (DE-OS 2 642 982; J. Org. Chem. 30, 3217 (1965)). Die Trifluormethoxyphenole der Formel III können ebenfalls nach literaturbekannten Verfahren hergestellt werden (US-PS 3 265 741; US-PS 4 157 344; Z. obsc. Chim. 31, 915—924 (1961)).

Folgende weitere Verfahren führen ebenfalls zu Verbindungen der Formel I:

Trifluormethoxyphenyl-thiophosphorsäureester der Formel Ia lassen sich in einer Arbusow-Reaktion durch Umsetzung von Phosphorigsäureestern der Formel IV mit Sulfenylchloriden der Formel $R^2SCl$ nach folgendem Reaktionsschema herstellen:

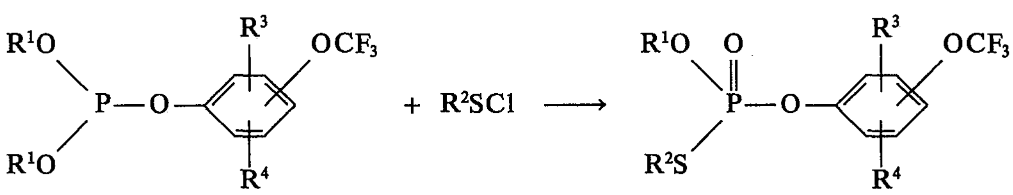

(IV) (Ia)

Ebenso sind die Trifluormethoxyphenyl-thiophosphorsäureester der Formel Ia durch Alkylierung von Phosphorsäureestersalzen der Formel V mit Alkylierungsmitteln der Formel $R^2Y$ zugänglich:

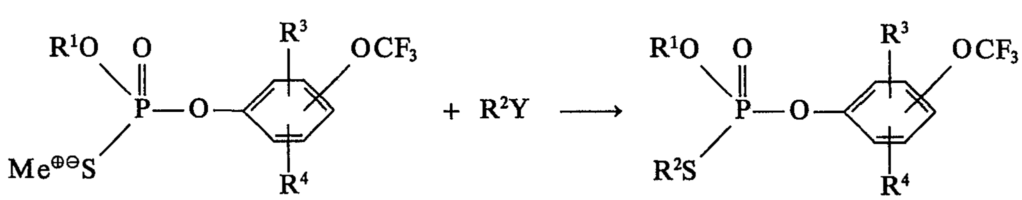

(V) (Ia)

Außerdem lassen sich Phosphorsäureesterdichloride der Formel VI mit Alkoholen und Mercaptanen der Formeln $R^1OH$ bzw. $R^2SH$ zu Verbindungen der Formel I umsetzen:

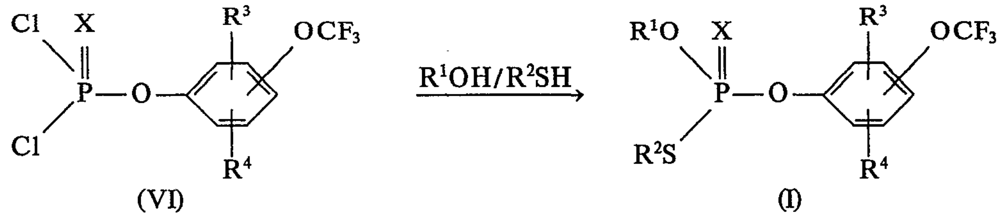

(VI) (I)

In diesen Reaktionsschemata haben die Substituenten $R^1$, $R^2$, $R^3$ und $R^4$ sowie X die obengenannten Bedeutungen. $Me^{\oplus}$ steht für ein Metallkation oder ein gegebenenfalls substituiertes Ammonium und Y bedeutet Halogenid, beispielsweise Jodid, Bromid, Chlorid, oder Alkylsulfat, beispielsweise Methylsulfat.

Herstellungsbeispiel

Zu einer Lösung von 8,9 Gewichtsteilen 4-Trifluormethoxyphenol in 30 Gewichtsteilen Eisessig gibt man 0,05 Gewichtsteile Diphenylsulfid und tropft dann ohne Kühlung 6,8 Gewichtsteile Sulfurylchlorid zu. Man rührt 3 Stunden bei Raumtemperatur nach, zieht das Lösungsmittel ab und nimmt den Rückstand in 50 Gewichtsteilen Methylenchlorid auf. Man wäscht diese Lösung mit 50 Gewichtsteilen gesättigter Hydrogencarbonatlösung, trocknet und engt ein. Die Destillation unter vermindertem Druck ergibt 9,8 Gewichtsteile (92% d. Th.) 2-Chlor-4-trifluormethoxyphenol vom Siedepunkt 70°C/ 13 mbar.

Zu 7,0 Gewichtsteilen 2-Chlor-4-trifluormethoxyphenol in 100 Gewichtsteilen Acetonitril gibt man 4,6 Gewichtsteile Kaliumcarbonat und erwärmt unter Rühren eine Stunde auf Rückfluß. Dann tropft man bei 50°C 6,5 Gewichtsteile O-Ethyl-S-sec-butylthiophosphoresterchlorid zu und rührt 2 Stunden bei 50°C und anschließend bei Raumtemperatur. Man entfernt das Lösungsmittel am Rotationsverdampfer, versetzt mit 300 Gewichtsteilen Toluol und 100 Gewichtsteilen Wasser, trennt die Phasen und wäscht die organische Phase mit 2 n Natronlauge und anschließend mit Wasser, trocknet mit Natriumsulfat und entfernt das Lösungsmittel und flüchtige Verunreinigungen unter vermindertem Druck bei 40°C und 0,1 mbar. Man erhält als Rückstand 8,7 Gewichtsteile O-Ethyl-S-sec.-butyl-O-(2-chloro-4-trifluormethoxyphenyl)-thiophosphat $n_D^{23} = 1{,}4795$.

Folgende Verbindungen der Formel I lassen sich beispielsweise analog oder nach einem der oben beschriebenen Verfahren herstellen:

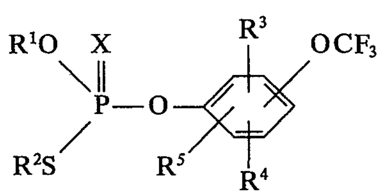

| Nr. | $R^1$ | $R^2$ | X | Position von $OCF_3$ | $R^3$ | $R^4$ | $R^5$ | $n_D$ |
|---|---|---|---|---|---|---|---|---|
| 1 | $CH_3$ | n-$C_3H_7$ | S | 2 | H | H | H | |
| 2 | $CH_3$ | n-$C_3H_7$ | O | 3 | H | H | H | |
| 3 | $CH_3$ | n-$C_3H_7$ | S | 4 | H | H | H | |
| 4 | $CH_3$ | n-$C_3H_7$ | O | 2 | H | H | H | |
| 5 | $CH_3$ | n-$C_3H_7$ | O | 4 | H | H | H | |
| 6 | $CH_3$ | sec-$C_4H_9$ | S | 2 | H | H | H | |
| 7 | $CH_3$ | sec-$C_4H_9$ | S | 3 | H | H | H | |
| 8 | $CH_3$ | sec-$C_4H_9$ | S | 4 | H | H | H | |
| 9 | $CH_3$ | sec-$C_4H_9$ | O | 2 | H | H | H | |
| 10 | $CH_3$ | sec-$C_4H_9$ | O | 3 | H | H | H | |
| 11 | $CH_3$ | sec-$C_4H_9$ | O | 4 | H | H | H | |
| 12 | $CH_3$ | n-$C_3H_7$ | O | 2 | H | 4-Cl | | |
| 13 | $CH_3$ | n-$C_3H_7$ | O | 3 | H | 4-Cl | | |
| 14 | $CH_3$ | n-$C_3H_7$ | O | 4 | H | 2-Cl | | |
| 15 | $CH_3$ | n-$C_3H_7$ | O | 2 | H | 4-Br | | |
| 16 | $CH_3$ | n-$C_3H_7$ | O | 3 | H | 4-Br | | |
| 17 | $CH_3$ | n-$C_3H_7$ | O | 4 | H | 2-Br | | |
| 18 | $CH_3$ | sec-$C_4H_9$ | O | 2 | H | 4-Cl | | |
| 19 | $CH_3$ | sec-$C_4H_9$ | O | 3 | H | 4-Cl | | |
| 20 | $CH_3$ | sec-$C_4H_9$ | O | 4 | H | 2-Cl | | |
| 21 | $C_2H_5$ | n-$C_3H_7$ | O | 2 | H | H | | |
| 22 | $C_2H_5$ | n-$C_3H_7$ | O | 4 | H | H | | |
| 23 | $C_2H_5$ | n-$C_3H_7$ | O | 2 | H | 4-Cl | | |
| 24 | $C_2H_5$ | n-$C_3H_7$ | O | 3 | H | H | | $n_D^{27} = 1.4670$ |
| 25 | $C_2H_5$ | n-$C_3H_7$ | O | 4 | H | H | | |
| 26 | $C_2H_5$ | n-$C_3H_7$ | O | 2 | H | 4-Cl | | |
| 27 | $C_2H_5$ | n-$C_3H_7$ | O | 3 | H | 4-Cl | | |
| 28 | $C_2H_5$ | n-$C_3H_7$ | O | 4 | H | 2-Cl | | $n_D^{25} = 1.4795$ |
| 29 | $C_2H_5$ | n-$C_3H_7$ | O | 2 | H | 4-Br | | |
| 30 | $C_2H_5$ | n-$C_3H_7$ | O | 3 | H | 4-Br | | |

| Nr. | $R^1$ | $R^2$ | X | Position von $OCF_3$ | $R^3$ | $R^4$ | $R^5$ | $n_D$ |
|---|---|---|---|---|---|---|---|---|
| 31 | $C_2H_5$ | n-$C_3H_7$ | O | 4 | H | H | | |
| 32 | $C_2H_5$ | n-$C_3H_7$ | O | 2 | 6-Cl | 4-Cl | | |
| 33 | $C_2H_5$ | n-$C_3H_7$ | O | 3 | 6-Cl | 4-Cl | | |
| 34 | $C_2H_5$ | n-$C_3H_7$ | O | 4 | 6-Cl | 4-Cl | | |
| 35 | $C_2H_5$ | n-$C_3H_7$ | O | 2 | 6-Br | 4-Br | | |
| 36 | $C_2H_5$ | n-$C_3H_7$ | O | 3 | 6-Br | 4-Br | | |
| 37 | $C_2H_5$ | n-$C_3H_7$ | O | 4 | 6-Br | H | | |
| 38 | $C_2H_5$ | n-$C_3H_7$ | O | 2 | H | 5-$CH_3$ | | |
| 39 | $C_2H_5$ | n-$C_3H_7$ | O | 3 | H | 5-$CH_3$ | | |
| 40 | $C_2H_5$ | n-$C_3H_7$ | O | 4 | H | 5-$CH_3$ | | |
| 41 | $C_2H_5$ | n-$C_3H_7$ | O | 2 | H | 4-CN | | |
| 42 | $C_2H_5$ | n-$C_3H_7$ | O | 3 | H | 4-CN | | |
| 43 | $C_2H_5$ | n-$C_3H_7$ | O | 4 | 3-CN | H | | |
| 44 | $C_2H_5$ | n-$C_3H_7$ | S | 2 | H | H | | |
| 45 | $C_2H_5$ | n-$C_3H_7$ | O | 3 | H | H | | $n_D^{26}$ =1.4975 |
| 46 | $C_2H_5$ | n-$C_3H_7$ | O | 4 | H | H | | |
| 47 | $C_2H_5$ | n-$C_3H_7$ | S | 2 | H | 4-Cl | | |
| 48 | $C_2H_5$ | n-$C_3H_7$ | S | 3 | H | 4-Cl | | |
| 49 | $C_2H_5$ | n-$C_3H_7$ | S | 4 | 2-Cl | H | | $n_D^{24}$ =1.5100 |
| 50 | $C_2H_5$ | n-$C_3H_7$ | S | 2 | H | 4-Br | | |
| 51 | $C_2H_5$ | n-$C_3H_7$ | S | 3 | H | 4-Br | | |
| 52 | $C_2H_5$ | n-$C_3H_7$ | S | 4 | 2-Br | H | | |
| 53 | $C_2H_5$ | i-$C_3H_7$ | O | 2 | H | H | | |
| 54 | $C_2H_5$ | i-$C_3H_7$ | O | 3 | H | H | | |
| 55 | $C_2H_5$ | i-$C_3H_7$ | O | 4 | H | H | | |
| 56 | $C_2H_5$ | i-$C_3H_7$ | O | 2 | H | 4-Cl | | |
| 57 | $C_2H_5$ | i-$C_3H_7$ | O | 3 | H | 4-Cl | | |
| 58 | $C_2H_5$ | i-$C_3H_7$ | O | 4 | 2-Cl | H | | |
| 59 | $C_2H_5$ | i-$C_3H_7$ | O | 2 | H | 4-Br | | |
| 60 | $C_2H_5$ | i-$C_3H_7$ | O | 3 | H | 4-Br | | |

| Nr. | $R^1$ | $R^2$ | X | Position von $OCF_3$ | $R^3$ | $R^4$ | $R^5$ | $n_D$ |
|---|---|---|---|---|---|---|---|---|
| 61 | $C_2H_5$ | i-$C_3H_7$ | O | 4 | H | 2-Cl | | |
| 62 | $C_2H_5$ | sec-$C_4H_9$ | O | 2 | H | H | | |
| 63 | $C_2H_5$ | sec-$C_4H_9$ | O | 3 | H | H | | $n_D^{26}$ = 1.4683 |
| 64 | $C_2H_5$ | sec-$C_4H_9$ | O | 4 | H | H | | |
| 65 | $C_2H_5$ | sec-$C_4H_9$ | O | 2 | H | 4-Cl | | |
| 66 | $C_2H_5$ | i-$C_3H_7$ | O | 3 | H | 4-Cl | | |
| 67 | $C_2H_5$ | i-$C_3H_7$ | O | 2 | H | 4-Br | | |
| 68 | $C_2H_5$ | i-$C_3H_7$ | O | 3 | H | 4-Br | | |
| 69 | $C_2H_5$ | i-$C_3H_7$ | O | 4 | 2-Br | H | | |
| 70 | $C_2H_5$ | i-$C_3H_7$ | O | 2 | 6-Cl | 4-Cl | | |
| 71 | $C_2H_5$ | i-$C_3H_7$ | O | 3 | 6-Cl | 4-Cl | | |
| 72 | $C_2H_5$ | i-$C_3H_7$ | O | 4 | 2-Cl | 6-Cl | | |
| 73 | $C_2H_5$ | i-$C_3H_7$ | O | 2 | 6-Br | 4-Br | | |
| 74 | $C_2H_5$ | i-$C_3H_7$ | O | 3 | 6-Br | 4-Br | | |
| 75 | $C_2H_5$ | i-$C_3H_7$ | O | 4 | 2-Br | 6-Br | | |
| 76 | $C_2H_5$ | i-$C_3H_7$ | O | 2 | H | 4-CN | | |
| 77 | $C_2H_5$ | i-$C_3H_7$ | O | 3 | 2-CN | H | | |
| 78 | $C_2H_5$ | i-$C_3H_7$ | O | 4 | H | 4-CN | | |
| 79 | $C_2H_5$ | i-$C_3H_7$ | O | 2 | H | 5-$CH_3$ | | |
| 80 | $C_2H_5$ | sec-$C_4H_9$ | O | 3 | H | 5-$CH_3$ | | |
| 81 | $C_2H_5$ | sec-$C_4H_9$ | O | 4 | H | 5-$CH_3$ | | |
| 82 | $C_2H_5$ | sec-$C_4H_9$ | O | 2 | H | 4-F | | |
| 83 | $C_2H_5$ | sec-$C_4H_9$ | O | 3 | H | 4-F | | |
| 84 | $C_2H_5$ | sec-$C_4H_9$ | O | 4 | 2-F | H | | |
| 85 | $C_2H_5$ | sec-$C_4H_9$ | S | 2 | H | H | | |
| 86 | $C_2H_5$ | sec-$C_4H_9$ | S | 3 | H | H | | |
| 87 | $C_2H_5$ | sec-$C_4H_9$ | S | 4 | H | H | | |
| 88 | $C_2H_5$ | sec-$C_4H_9$ | S | 2 | H | 4-Cl | | |
| 89 | $C_2H_5$ | sec-$C_4H_9$ | S | 3 | H | 4-Cl | | |
| 90 | $C_2H_5$ | sec-$C_4H_9$ | S | 4 | 2-Cl | H | | |

| Nr. | $R^1$ | $R^2$ | X | Position von $OCF_3$ | $R^3$ | $R^4$ | $R^5$ | $n_D$ |
|---|---|---|---|---|---|---|---|---|
| 91 | $C_2H_5$ | i-$C_4H_9$ | S | 2 | H | H | | |
| 92 | $C_2H_5$ | i-$C_4H_9$ | O | 3 | H | H | | |
| 93 | $C_2H_5$ | i-$C_4H_9$ | O | 4 | H | H | | |
| 94 | $C_2H_5$ | i-$C_4H_9$ | O | 2 | H | 4-Cl | | |
| 95 | $C_2H_5$ | i-$C_4H_9$ | O | 3 | H | 4-Cl | | |
| 96 | $C_2H_5$ | i-$C_4H_9$ | O | 4 | 2-Cl | H | | $n_D^{23}$ = 1.4780 |
| 97 | $C_2H_5$ | i-$C_4H_9$ | O | 2 | H | 4-Br | | |
| 98 | $C_2H_5$ | i-$C_4H_9$ | O | 3 | | 4-Br | | |
| 99 | $C_2H_5$ | i-$C_4H_9$ | O | 4 | 2-Br | H | | |
| 100 | $C_2H_5$ | i-$C_4H_9$ | S | 2 | H | H | | |
| 101 | $C_2H_5$ | i-$C_4H_9$ | S | 3 | H | H | | |
| 102 | $C_2H_5$ | i-$C_4H_9$ | S | 4 | H | H | | |
| 103 | $C_2H_5$ | i-$C_4H_9$ | S | 2 | H | 4-Cl | | |
| 104 | $C_2H_5$ | i-$C_4H_9$ | S | 3 | H | 4-Cl | | |
| 105 | $C_2H_5$ | i-$C_4H_9$ | S | 4 | 2-Cl | H | | |
| 106 | $C_2H_5$ | $CH_3O-(CH_2)_2-$ | O | 2 | H | H | | |
| 107 | $C_2H_5$ | $CH_3O-(CH_2)_2-$ | O | 3 | H | H | | |
| 108 | $C_2H_5$ | $CH_3O-(CH_2)_2-$ | O | 4 | H | H | | |
| 109 | $C_2H_5$ | $CH_3O-(CH_2)_2-$ | O | 2 | H | 4-Cl | | |
| 110 | $C_2H_5$ | $CH_3O-(CH_2)_2-$ | O | 3 | H | 4-Cl | | |
| 111 | $C_2H_5$ | $CH_3O-(CH_2)_2-$ | O | 4 | 2-Cl | 4 | | |
| 112 | $C_2H_5$ | $CH_3O-(CH_2)_2-$ | O | 2 | H | 4-Br | | |
| 113 | $C_2H_5$ | $CH_3O-(CH_2)_2-$ | O | 3 | H | 4-Br | | |
| 114 | $C_2H_5$ | $CH_3O-(CH_2)_2-$ | O | 4 | 2-Br | H | | |
| 115 | $C_2H_5$ | $C_2H_5O-(CH_2)_2-$ | O | 2 | H | H | | |
| 116 | $C_2H_5$ | $C_2H_5O-(CH_2)_2-$ | O | 3 | H | H | | |
| 117 | $C_2H_5$ | $C_2H_5O-(CH_2)_2-$ | O | 4 | H | H | | |
| 118 | $C_2H_5$ | $C_2H_5O-(CH_2)_2-$ | O | 2 | H | 4-Cl | | |
| 119 | $C_2H_5$ | $C_2H_5O-(CH_2)_2-$ | O | 3 | H | 4-Cl | | |
| 120 | $C_2H_5$ | $C_2H_5O-(CH_2)_2-$ | O | 4 | H | 2-Cl | | |

| Nr. | $R^1$ | $R^2$ | X | Position von $OCF_3$ | $R^3$ | $R^4$ | $R^5$ | $n_D$ |
|---|---|---|---|---|---|---|---|---|
| 121 | $C_2H_5$ | $C_2H_5O-(CH_2)_2-$ | O | 2 | H | 4-Br | | |
| 122 | $C_2H_5$ | $C_2H_5O-(CH_2)_2-$ | O | 3 | H | 4-Br | | |
| 123 | $C_2H_5$ | $C_2H_5O-(CH_2)_2-$ | O | 4 | 2-Br | H | | |
| 124 | $C_2H_5$ | i-$C_3H_7O-(CH_2)_2-$ | O | 2 | H | H | | |
| 125 | $C_2H_5$ | i-$C_3H_7O-(CH_2)_2-$ | O | 3 | H | H | | |
| 126 | $C_2H_5$ | i-$C_3H_7O-(CH_2)_2-$ | O | 4 | H | H | | |
| 127 | $C_2H_5$ | i-$C_3H_7O-(CH_2)_2-$ | O | 2 | H | 4-Cl | | |
| 128 | $C_2H_5$ | i-$C_3H_7O-(CH_2)_2-$ | O | 3 | H | 4-Cl | | |
| 129 | $C_2H_5$ | i-$C_3H_7O-(CH_2)_2-$ | O | 4 | 2-Cl | H | | |
| 130 | $C_2H_5$ | i-$C_3H_7O-(CH_2)_2-$ | O | 2 | H | 4-Br | | |
| 131 | $C_2H_5$ | i-$C_3H_7O-(CH_2)_2-$ | O | 3 | H | 4-Br | | |
| 132 | $C_2H_5$ | i-$C_3H_7O-(CH_2)_2-$ | O | 4 | 2-Br | H | | |
| 133 | $C_2H_5$ | $CH_3-(CH_2)_2-CH(CH_3)-$ | O | 2 | H | H | | |
| 134 | $C_2H_5$ | $CH_3-(CH_2)_2-CH(CH_3)-$ | O | 3 | H | H | | |
| 135 | $C_2H_5$ | $CH_3-(CH_2)_2-CH(CH_3)-$ | O | 4 | H | H | | |
| 136 | $C_2H_5$ | $C_5H_9$ | O | 2 | H | H | | |
| 137 | $C_2H_5$ | $C_5H_9$ | O | 3 | H | H | | |
| 138 | $C_2H_5$ | $C_5H_9$ | O | 4 | H | H | | |
| 139 | $C_2H_5$ | $ClCH_2-(CH_2)_2-$ | O | 2 | H | H | | |
| 140 | $C_2H_5$ | $ClCH_2-(CH_2)_2-$ | O | 3 | H | H | | |
| 141 | $C_2H_5$ | $ClCH_2-(CH_2)_2-$ | O | 4 | H | H | | |

Die erfindungsgemäßen Trifluormethoxyphenyl-(di)thiophosphorsäureester der Formel I sind geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören

aus der Ordnung der Schmetterlinge (Lepidoptera) beispielsweise Plutella maculipennis (Kohlschabe), Leucoptera coffeella (Kaffeemotte), Hyponomeuta malinellus (Apfelbaumgespinstmotte), Argyresthia conjugella (Apfelmotte), Sitotroga cerealella (Getreibemotte), Phthorimaea operculella (Kartoffelmotte), Capua reticulana (Apfelschalenwickler), Sparganothis pilleriana (Sprungwurm), Cacoecia murinana (Tannentriebwickler), tortrix viridana (Eichenwickler), Clysia ambiguella (Heu- und Sauerwurm), Evetria buoliana (Kieferntriebwickler), Polychrosis botrana (Bekreuzter Traubenwickler), Cydia pomonella (Obstmade), Laspeyresia molesta (Pfirsichtriebbohrer), Laspeyresia funebrana (Pflaumenwickler), Ostrinia nubilalis (Maiszünsler), Loxostege sticticalis (Rübenzünsler), Ephestia kuehniella (Mehlmotte), Chilo suppressalis (Reisstengelbohrer), Galleria Mellonella (Wachsmotte), Malacosoma neustria (Ringelspinner), Dendrolimus pini (Kiefernspinner), Thaumatopoea pityocampa (Pinienprozessionsspinner), Phalera bucephala (Mondfleck), Cheimatobia brumata (Kleiner Frostspanner), Hibernia defoliaria (Großer Frostspanner), Bupalus piniarus (Kiefernspanner), Hypantria cunea (Weißer Bärenspinner), Agrotis segetum (Wintersaateule), Agrotis ypsilon (Ypsiloneule), Barathra brassicae (Kohleule), Cirphis unipuncta (Heerwurm), Prodenia litura (Baumwollraupe), Laphygma exigua (Rüben-Heerwurm), Panolis flammea (Forleule), Earias insulana (Baumwollkapselwurm), Plusia gamma (Gammaeule), Alabama argillacea (Baumwollblattwurm), Lymantriadispar (Schwammspinner), Lymantria monacha (Nonne), Pieris brassicae (Kohlweißling), Aporia crataegi (Baumweißling);
aus der Ordnung der Käfer (Coleoptera) beispielsweise Blitophaga undata (Schwarzer Rübenaaskäfer), Melanotus communis (Drahtwurm), Limonius californicus (Drahtwurm), Agriotes lineatus (Saatschnellkäfer), Agricotes obscurus (Humusschnellkäfer), Agrilus sinuatus (Birnbaum-Prachtkäfer), Meligethes aeneus (Rapsglanzkäfer), Atomaria linearis (Moosknopfkäfer), Epilachna varicestris (Mexikanischer Bohnenkäfer), Phyllopertha horticola (Junikäfer), Popillia japonica (Japankäfer), Melolontha melolontha (Feldmaikäfer), Melolontha hippocastani (Waldmaikäfer), Amphimallus solstitialis (Brachkäfer), Crioceris asparagi (Spargelhähnchen), Lema melanopus (Getreidehähnchen), Leptinotarsa decemlineata (Kartoffelkäfer), Phaedon cochleariae (Meerrettich-Blattkäfer), Phyllotreta nemorum (Kohlerdfloh), Chaetcnema tibialis (Rübenflohkäfer), Phylloides chrysocephala (Raps-Flohkäfer), Diabrotica 12-punctata (Südlicher Maiswurzelwurm), Cassida nebulosa (Nebliger Schildkäfer), Bruchus lentis (Linsenkäfer), Bruchus rufimanus (Pferdebohnenkäfer), Bruchus pisorum (Erbsenkäfer), Sitona lineatus (Linierter Blattrandkäfer), Otiorrhynchus sulcatus (Gefurchter Lappenrüßler), Otiorrhynchus ovatus (Erdbeerwurzelrüßler), Hylobies abietis (Großer Brauner Rüsselkäfer), Byctiscus betulae (Rebenstecher), Anthonomus pomorum (Apfelblütenstecher), Anthonomus grandis (Kapselkäfer), Ceuthorrhynchus assimilis (Kohlschotenrüßler), Ceuthorrhynchus napi (Großer Kohltriebrüßler), Sitophilus granaria (Kornkäfer), Anisandrus dispar (Ungleicher Holzborkenkäfer), Ips typographus (Buchdrucker), Blastophagus piniperda (Gefurchter Waldgärtner);
aus der Ordnung der Zweiflügler (Diptera) beispielsweise Lycoria pectoralis, Mayetiola destructor (Hessenfliege), Dasineura brassicae (Kohlschoten-Gallmücke), Contarinia tritici (Gelbe Weizen-Gallmücke), Haplodiplosis equestris (Sattelmücke), Tipula paludosa (Wiesenschnake), Tipula oleracea (Kohlschnake), Dacus cucurbitae (Melonenfliege), Dacus oleae (Olivenfliege), Ceratitis capitata (Mittelmeerfruchtfliege), Rhagoletis cerasi (Kirschfruchtfliege), Rhagoletis pomonella (Apfelmade), Anastrepha ludens (Mexikanische Fruchtfliege), Oscinella frit (Fritfliege), Phorbia coarctata (Brachfliege), Phorbia antiqua (Zwiebelfliege), Phorbia brassicae (Kleine Kohlfliege), Pegomya hyoscyami (Rübenfliege), Anopheles maculipennis, Culex pipiens, Aedes aegypti (Gelbfiebermücke), Aedes vexans, Tabanus bovinus (Rinderbremse), Tipula paludosa (Wiesenschnake), Musca domestica (Stubenfliege), Fannia canicularis (Kleine Stubenfliege), Muscina stabulans, Glossina morsitans (Tsetse-Fliege), Oestrus ocis, Chrysomya macellaria, Chrysomya hominivorax, Lucilia cuprina, Lucilia sericata, Hypoderma lineata;
aus der Ordnung der Hautflügler (Hymenoptera) beispielsweise Athalia rosae (Rübenblattwespe), Hoplocampa minuta (Pflaumensägewespe), Monomorium pharaonis (Pharaoameise), Solenopsis geminata (Feuerameise), atta sexdens (Blattschneiderameise);
aus der Ordnung der Wanzen (Heteroptera) beispielsweise Nezara viridula (Grüne Reiswanze), Eurygaster integriceps (Asiatische Getreidewanze), Blissus leucopterus (Chinch bug), Dysdercus cingulatus (Kapok-Wanze), Dysdercus intermedius (Baumwollwanze), Piesma quadrata (Rübenwanze), Lygus pratensis (Gemeine Wiesenwanze);
aus der Ordnung der Pflanzensauger (Homoptera) beispielsweise Perkinsiella saccharicida (Zuckerrohrzikade), Nilaparvata lugens (Braune Zikade), Empoasca fabae (Kartoffelzikade), Psylla mali (Apfelblattsauger), Psylla piri (Birnblattsauger), Trialeurodes vaporariorum (Weiße Fliege), Aphis fabae (Schwarze Bohnenlaus), Aphis pomi (Grüne Apfellaus), Aphis sambuci (Holunderblattlaus), aphidula nastrutii (Kreuzdornblattlaus), Cerosipha gossypii (Gurkenblattaus), Sappaphis mali (Rosige Apfellaus), Sappaphis mala (Mehlige Birnblattlaus), Dysphis radicola (Mehlige Apfelfalterlaus), Brachycaudus cardui (Große Pflaumenblattlaus), Brevicoryne brassicae (Kohlblattlaus), Phorodon humuli (Hopfenblattlaus), Rhopalomyzus ascalonicus (Zwiebellaus), Myzodes persicae (Grüne Pfirsichlaus), Myzus cerasi (Schwarze Sauerkirschenlaus), Dysaulacorthum pseudosolani (Gefleckte Kartoffellaus), Acyrthosiphon onobrychis (Grüne Erbsenlaus), Macrosiphon rasae (Große Rosenblattlaus), Megoura viciae (Wickenlaus), Schizoneura lanuginosa (Birnenblattlaus), Pemphigus bursarius (Salatwurzellaus), Dreyfusia nordmannianae (Tannentrieblaus), Dreyfusia piceae (Weißtannenstammlaus), Adelges laricis (Rote Fichtengallenlaus), Viteus vitifolii (Reblaus);
aus der Ordnung der Termiten (Isoptera) beispielsweise Reticulitermes lucifugus,Calotermes flavicollis, Leucotermes flavipes, Termes natalensis;
aus der Ordnung der Geradflügler (Orthoptera) beispielsweise Forficula auricularia (Gemeiner Ohrwurm), Acheta domestica (Heimchen), Gryllotalpa (Maulwurfsgrille), Tachycines asynamorus (Gewächshausschrecke), Locusta migratoria (Wanderheuschrecke), Stauronotus maroccanus (Marokkanische Wanderheuschrecke), Schistocerca peregrina (Wanderheuschrecke), Nomadacris septemfasciata (Wanderheuschrecke), Melanoplus spretus (Felsengebirgsheuschrecke), Melano plus femur-rubrum (Rotbeinige Heuschrecke), Blatta orientalis (Küchenschabe), Blattella

germanica (Deutsche Schabe), Periplaneta americana (Amerikanische Schabe), Blabera gigantea (Riesenschabe).

Zur Klasse der Arachnoidea gehören Spinnentiere (Acarina), beispielsweise Ixodes ricinus (Holzbock), Ornithodorus moubata, Amblyomma americanum, Dermacentor silvarum, Boophilus microplus, Tetranychus telarius, Tetranychus atlanticus, Tetranychus pacificus, Paratetranychus pilosus, Bryobia praetiosa.

Zur Klasse der Nemathelminthes zählen beispielsweise Wurzelgallennematoden, z. B. Meloidogyne incognita, Meloidogyne hapla, Meloidogyne javanica, Zysten bildende Nematoden, z. B. Heterodera rostochiensis, Heterodera schachtii, Heterodera avenae, Heterodera glycines, Heterodera triflolii, Stock- und Blattälchen, z. B. Ditylenchus dipsaci, Ditylenchus destructor, Pratylenchus neglectus, Pratylenchus penetrans, Partylenchus goodeyi, Paratylenchus curvitatus sowie Tylenchorhynchus dubius, Tylenchorhynchus claytoni, Rotylenchus robustus, Heliocotylenchus multicinctus, Radopholus similis, Belonolaimus longicaudatus, Longidorus elongatus, Trichodorus primitivus.

Die Wirkstoffe können als solche, in Form ihrer Formulierungen oder den daraus bereiteten Anwendungsmöglichkeiten, z. B. in Form von direkt versprühbaren Lösungen, Pulvern, Suspensionen oder Dispersionen, Emulsionen, Öldispersionen, Pasten, Stäubemitteln, Streumitteln, Granulaten duch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z. B. Benzol, Toluol, Xylol, Paraffin, Tetrahydronaphthalin, alkylierte Naphthaline oder deren Derivate, z. B. Methanol, Ethano, Propanol, Butanol, Chloroform, Tetrachlorkohlenstoff, Cyclohexanol, Cyclohexanon, Chlorbenzol, Isophoron, stark polare Lösungsmittel, z. B. Dimethylformamid, Dimethylsulfoxid, N-Methylpyrrolidon, Wasser, in Betracht.

Wäßrige Anwendungsformen können aus Emulsionskonzentraten, Pasten oder netzbaren (Spritzpulvern, Öldispersionen) durch Zusatz von Wasser bereitet werden. Zur Herstellung von Emulsionen, Pasten oder Öldispersionen können die Substanzen als solche in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkaly-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Nonylphenol, Alkylphenolpolyglykolether, Tributylphenylpolyglykolether, Alkylarylpolyetheralkohole, Isotridecylalkohol,Fettalkoholethylenoxid-Kondensate, ethoxyliertes Rizinusöl, Polyoxyethylenalkylether, ethoxyliertes Polyoxyproylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate, können durch Bindung der Wirkstoffe an feste Trägerstoffe hergestellt werden. Feste Trägerstoffe sind z. B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,1 und 95 Gew.-% Wirkstoff, vorzugsweise zwischen 0,5 und 90 Gew.-%.

Die Wirkstoffkonzentrationen in den anwendungsfertigen Zubereitungen können in größeren Bereichen variiert werden. Im allgemeinen liegen sie zwischen 0,0001 und 10%, vorzugsweise zwischen 0,01 und 1%.

Beispiele für Formulierungen sind:

I. 5 Gewichtsteile der Verbindung Nr. 17 werden mit 95 Gewichtsteilen feinteiligem Kaolin innig vermischt. Man erhält auf diese Weise ein Stäubemittel, das 3 Gew.-% des Wirkstoffs enthält.

II. 30 Gewichtsteile der Verbindung Nr. 24 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des

Wirksstoffs mit guter Haftfähigkeit.

III. 10 Gewichtsteile der Verbindung Nr. 28 werden in einer Mischung gelöst, die aus 90 Gewichtsteilen Xylol, 6 Gewichtsteilen des Anlagerungsproduktes von 8 bis 10 Mol Äthylenoxid an 1 Mol Ölsäure-N-monoethanolamid, 2 Gewichtsteilen Calciumsalz der Dodecylbenzolsulfonsäure und 2 Gewichtsteilen des Anlagerungsproduktes von 40 Mol Ethylenoxid an 1 Mol Ricinusöl besteht.

IV. 20 Gewichtsteile der Verbindung Nr. 48 werden in einer Mischung gelöst, die aus 60 Gewichtsteilen Cyclohexanon, 30 Gewichtsteilen Isobutanol, 5 Gewichtsteilen des Anlagerungsproduktes von 7 Mol Ethylenoxid an 1 Mol Isooctylphenol und 5 Gewichtsteilen des Anlagerungsproduktes von 40 Mol Ethylenoxid an 1 Mol Ricinusöl besteht.

V. 80 Gewichtsteile der Verbindung Nr. 45 werden mit 3 Gewichtsteilen des Natriumsalzes der Diisobutylnaphthalin-alpha-sulfonsäure, 10 Gewichtsteilen des Natriumsalzes einer Ligninsulfonsäure aus einer Sulfit-Ablauge und 7 Gewichtsteilen pulverförmigem Kieselsäuregel gut vermischt und in einer Hammermühle vermahlen.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Die Aufwandmenge an Wirkstoff beträgt unter Freilandbedingungen 0,2 bis 10 kg/ha, vorzugsweise 0,5 bis 2,0 kg/ha.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1 : 10 bis 10 : 1 zugemischt werden.

Beispielsweise können folgende Mittel zugemischt werden:

1,2-Dibrom-3-chlorpropan, 1,3-Dichlorpropen, 1,3-Dichlorpropen + 1,2-Dichlorproppan,
1,2-Dibrom-ethan, 2-sec.-Butyl-phenyl-N-methylcarbamat, o-Chlorphenyl-N-methylcarbamat,
3-Isopropyl-5-methylphenyl-N-methylcarbamat, o-Isopropoxyphenyl-N-methylcarbamat,
3,5-Dimethyl-4-methylmercapto-phenyl-N-methylcarbamat,
4-Dimethylamino-3,5-xylyl-N-methylcarbamat, 2-(1,3-Dioxolan-2-yl)-phenyl-N-methylcarbamat,
I-Naphthyl-N-methylcarbamat, 2,3-Dihydro-2,2-dimethyl-benzofuran-7-yl-N-methylcarbamat,
2,2-Dimethyl-1,3-benzodioxol-4-yl-N-methylcarbamat,
2-Dimethylamino-5,6-dimethyl-4-pyrimidinyl-dimethylcarbamat,
2-Methyl-2-(methylthio)-propionaldehyd-O-(methylcarbamoyl)-oxim,
S-Methyl-N-[(methylcarbamoyl)-oxy]-thio-acetimidat,
Methyl-N',N'-dimethyl-N-[(methylcarbamoyl)oxy]-1-thioxamidat,
N-(2-Methyl-chlor-phenyl)-N',N'-dimethylformamidin, Tetrachlorthiophen,
1-(2,6-Difluor-benzoyl)-3-(4-chlor-phenyl)-harnstoff,
O,O-Dimethyl-O-(p-nitrophenyl)-phosphorthioat, O,O-Diethyl-O-(p-nitrophenyl)-phosphorthioat,
O-Ethyl-O-(p-nitrophenyl)-phenyl-phosphonothioat,
O,O-Dimethyl-O-(3-methyl-4-nitrophenyl)-phosphorthioat,
O,O-Diethyl-O-(2,4-dichlorphenyl)-phosphorthioat,
O-Ethyl-O-(2,4-dichlorphenyl)-phenyl-phosphonothioat,
O,O-Dimethyl-O-(2,4,5-trichlorphenyl)-phosphorthioat,
O-Ethyl-O-(2,4,5-trichlorphenyl)-ethyl-phosphonothioat,
O,O-Dimethyl-O-(4-brom-2,5-dichlorphenyl)-phosphorthioat,
O,O-Dimethyl-O-(2,5-dichlor-4-jodphenyl)-phosphorthioat,
O,O-Dimethyl-O-(e-methyl-4-methylthiophenyl)-phosphorthioat,
O-Ethyl-O-(3-methyl-4-methylthiophenyl)-isopropyl-phosphoramidat,
O,O-Diethyl-O-[p-methylsulfinyl-phenyl]-phosphorthioat,
O-Ethyl-S-phenyl-ethyl-phosphonodithioat,
O,O-Diethyl-[2-chlor-1-(2,4-dichlorphenyl)-vinyl]-phosphat,
O,O-Dimethyl-[-2-chlor-1-(2,4,5-trichlorphenyl)]-vinyl-phosphat,
O,O-Dimethyl-S-(1'-phenyl)-ethylacetat-phosphordithioat,
Bis-(dimethylamino)-fluorphosphinoxid, Octamethyl- pyrophosphoramid,
O,O,O,O-Tetraethyldithio-pyrophosphat, S-Chlormethyl-O,O-diethyl-phosphordithioat,
O-Ethyl-S,S-dipropyl-phosphordithioat, O,O-Dimethyl-O-2,2-dichlorvinyl- phosphat,
O,O-Dimethyl-1,2-dibrom-2,2-dichlorethylphosphat,
O,O-Dimethyl-2,2,2-trichlor-1-hydroxy-ethylphosphonat,
O,O-Dimethyl-S-[1,2-biscarbethoxy-ethyl-(1)]-phosphordithioat,
O,O-Dimethyl-O-(1-methyl-2-carbmethoxy-vinyl)-phosphat,
O,O-Dimethyl-S-(N-methyl-carbamoyl-methyl)-phosphordithioat,
O,O-Dimethyl-S-(N-methyl-carbamoyl-methyl)-phosphorthioat,
O,O-Dimethyl-S-(N-methoxyethyl-carbamoyl-methyl)-phosphordithioat,
O,O-Dimethyl-S-(N-formyl-N-methyl-carbamoylmethyl-phosphordithioat,

O,O-Dimethyl-O-[1-methyl-2-(methyl-carbamoyl)-vinyl]-phosphat,
O,O-Dimethyl-O-[(1-methyl-2-dimethylcarbamoyl)-vinyl]-phosphat,
O,O-Dimethyl-O-[(1-methyl-2-chlor-2-diethylcarbamoyl)-vinyl]-phosphat,
O,O-Diethyl-S-(ethylthio-methyl)-phosphordithioat,
O,O-Diethyl-S-[(p-chlorphenylthio)-methyl]-phosphordithioat,
O,O-Dimethyl-S-(2-ethylthioethyl)-phosphorthioat,
O,O-Dimethyl-S-(2-ethylthioethyl)-phosphordithioat,
O,O-Dimethyl-S-(2-ethylsulfinyl-ethyl)-phosphorthioat,
O,O-Diethyl-S-(2-ethylthio-ethyl)-phosphordithioat,
O,O-Diethyl-S-(2-ethylsulfinyl-ethyl)-phosphorthioat,
O,O-Diethyl-thiophosphoryliminophenyl-acetonitril,
O,O-Diethyl-S-(2-chlor-1-phthalimidoethyl)-phosphordithioat,
O,O-Diethyl-S-[6-chlor-benzoxazolon-(2)-yl(3)]-methyldithiophosphat,
O,O-Dimethyl-S-[2-methoxy-1,3,4-thiadiazol-5-[4H]-onyl-(4)-methyl]phosphordithioat,
O,O-Diethyl-O-[3,5,6-trichlor-pyridyl-(2)]-phosphorthioat,
O,O-Diethyl-O-(2-pyrazinyl)-phosphorthioat,
O,O-Diethyl-O-[2-isopropyl-4-methyl-pyrimidinyl(6)-phosphorthioat,
O,O-Diethyl-O-[2-diethylamino)-6-methyl-4-pyrimidinyl]-thionophosphat,
O,O-Dimethyl-S-(4-oxo-1,2,3-benzotriazin-3-[4H]-yl-methyl)-phosphordithioat,
O,O-Dimethyl-S-[(4,6-diamino-1,3,5-triazin-2-yl)-methyl]-phosphordithioat,
O,O-Diethyl-(1-phenyl-1,2,4-triazol-3-yl)-thionophosphat, O,S-Dimethyl-phosphor-amido-thioat,
O,S-Dimethyl-N-acetyl-Phosphoramidothioat, $\gamma$-Hexachlorcyclohexan,
1,1-Di-(p-methoxyphenyl)-2,2,2-trichlor-ethan,
6,7,8,9,10,10-Hexachloro-1,5,5a,6,9,9a-hexahydro-6,9-methano-2,4,3-benzodioxa-thiepin-3-oxid,
Pyrethrine, DL-2-Allyl-3-methyl-cyclopenten-(2)-on-(1)-yl-(4)-DL-cis,-trans-chrysanthemat,
5-Benzyl-furyl-(3)-methyl-DL-cis,-trans-chrysanthemat, 3-Phenoxybenzyl($\pm$)-cis,
trans-2,2-di-methyl-3-(2,2-dichlorvinyl)-cyclopropancarboxylat,
$\alpha$-Cyano-3-phenoxybenzyl($\pm$)-cis,trans-2,2-dimethyl-3-(2,2-dichlorvinyl)-cyclopropancarboxylat,
(s)-$\alpha$-Cyano-3-phenoxybenzyl-cis(1R,3R)-2,2-dimethyl-3-(2,2-dibromvinyl)-cyclopropancarboxylat, 3,4,5,6-Tetrahydrophthalimidoethyl-DL-cis,trans-chrysanthemat,
2-Methyl-5-(2-propinyl)-3-furylmethyl-chrysanthemat,
($\alpha$-Cyano-3-phenoxybenzyl)-$\alpha$-isopropyl-4-chlorphenylacetat.

Die folgenden Beispiele belegen die biologische Wirkung der neuen Verbindungen. Die Numerierung der Wirkstoffe entspricht der der tabellarischen Auflistung.

## Beispiel 1

### Kontaktwirkung auf Stubenfliegen (Musca domestica); Dauerkontakt

Beide Teile einer Petrischale von 10 cm Durchmesser werden mit insgesamt 2 ml der acetonischen Wirkstofflösung ausgekleidet. Nach dem Verdunsten des Lösungsmittels (ca. 30 Minuten) bringt man je 20 4 Tage alte Fliegen in die Schalen. Die Mortalitätsrate wird nach 4 Stunden festgestellt.

In diesem Test wird mit den Wirkstoffen Nr. 24, 28, 45, 49, 63 eine sehr hohe Mortalitätsrate erzielt.

## Beispiel 2

### Fraß- und Kontaktwirkung auf Raupen der Kohlschabe (Plutella maculipennis)

Blätter von jungen Kohlpflanzen werden 3 Sekunden lang in die wäßrige Wirkstoffemulsion getaucht und nach kurzem Abtropfen auf einen angefeuchteten Filter in eine Petrischale gelegt. Das Blatt wird darauf mit 10 Raupen des 4. Stadiums belegt. Nach 48 Stunden beurteilt man die Wirkung.

Bei diesem Test zeigen z. B. die folgenden Wirkstoffe eine sehr gute Wirkung: Nr. 24, 28, 45, 49, 63, 96.

## Beispiel 3

### Kontaktwirkung auf Schaben (Blatta orientalis)

Der Boden eines 1-l-Einmachglases wird mit der acetonischen Lösung des Wirkstoffs behandelt. Nach dem Verdunsten des Lösungsmittels setzt man je Glas 5 adulte Schaben. Die Mortalitätsrate wird nach 48 Stunden bestimmt.

In diesem Test zeigen die Wirkstoffe Nr. 24, 28, 45, 49, 63, 96 eine ausgezeichnete Wirkung.

Beispiel 4

Kontaktwirkung auf Kornkäfer (Sitophilus granaria)

Petrischalen von 10 cm Durchmesser werden mit acetonischer Wirkstofflösung ausgekleidet. Nach dem Verdunsten des Lösungsmittels belegt man die Schalen mit 100 Kornkäfern. Nach 4 Stunden werden die Käfer in unbehandelte Gefäße überführt. Die Mortalitätsrate wird nach 24 Stunden ermittelt. Dabei wird festgestellt, wieviele Käfer in der Lage sind, nach diesem Zeitpunkt innerhalb 60 Minuten ein unbehandeltes Pappschälchen (Durchmesser 40 mm, Höhe 10 mm) zu verlassen.

In diesem Test zeigen die Wirkstoffe Nr. 24, 28, 45, 49, 63 eine sehr gute Wirkung.

Beispiel 5

Kontaktwirkung auf Zecken (Ornithodorus moubata)

Geprüft wird mit Zecken im 3. Larvenstadium. Dazu taucht man die Tiefe, die sich in einem Papierbeutel befinden, für 3 Sekunden in die Prüfemulsion. Die Beutel werden frei aufgehängt. Nach 48 Stunden wird die Wirkung auf die Zecken beurteilt.

In diesem Test zeigen die Wirkstoffe Nr. 24, 28, 63 eine sehr gute Wirkung.

Beispiel 6

Systemische Wirkung auf Raupen (Prodenia litura)

200 ml Quarzsand werden in 250-ml-Kunststoff-Becher in Paletten zu 8 Gefäßen gegeben. Man belegt jeden Becher mit 5 Maiskörnern — ca. 1 cm unter die Oberfläche. Darauf befeuchtet man mit je 50 ml Wasser und deckt mit einer passenden transparenten Kunststoffhaube ab. Nach 8 Tagen werden die Paletten abgedeckt, nach 10 Tagen erfolgt die Behandlung. Dabei gießt man 40 ml der wäßrigen Wirkstoffaufbereitung an die Pflanzen und deckt nach einem weiteren Tag mit 50 ml trockenem Quarzsand ab. Hierdurch soll ein Kontakt der Versuchstiere mit der behandelten Oberfläche vermieden werden.

Auf jeden Becher stellt man einen Plastik-Zylinder (Durchmesser 7 cm), belegt mit je 5 Raupen im 3. Larvenstadium und deckt den Zylinder mit einem Drahtgazedeckel ab.

Nach 4 Tagen beurteilt man Fraß und Mortalität in den Gefäßen.

In diesem Test zeigen die Wirkstoffe Nr. 24, 28, 45, 49, 63, 96 eine gute Wirkung.

Beispiel 7

Zuchtversuch mit Stubenfliegen (Musca domestica)

50 g eines Nährbodens aus

| | |
|---|---|
| 100 Teilen | Wasser |
| 10 Teilen | Bäckerhefe |
| 10 Teilen | Trockenmilch und |
| 1 Teil | Agar |

werden in warmem Zustand mit der wäßrigen Aufbereitung des Wirkstoffes gründlich durchmischt.

Nach Erkalten belegt man den Nährboden mit ca. 0,1 ml Fliegeneiern und beobachtet deren Entwicklung über eine Woche.

Die Versuchstemperatur liegt bei 20° C.

In diesem Test zeigen die Wirkstoffe Nr. 24, 28, 45, 49, 63, 96 eine gute Wirkung.

Beispiel 8

Wirkung gegen Nematoden (Ditylenchus dipsaci)

Frisch aus lebendem Pflanzengewebe extrahierte Nematoden (Ditylenchus dipsaci) werden in reinem Leitungswasser suspendiert. Derartigen Suspensionen setzt man die wäßrige Wirkstoffaufbereitung zu und beurteilt die Mortalität nach 4 und 24 Stunden.

In diesem Test zeigen die Wirkstoffe Nr. 24, 28, 45, 49, 63, 96 eine gute Wirkung.

**Patentansprüche für die Vertragsstaaten: BE, CH, DE, FR, GB, IT, LI, LU, NI, SE**

1. Trifluormethoxyphenyl-(di)thiophosphorsäureester der Formel

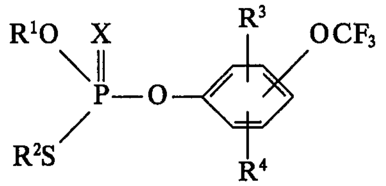

(I)

in der

- $R^1$ Alkyl mit 1 bis 3 Kohlenstoffatomen,
- $R^2$ Alkyl oder Halogenalkyl mit 1 bis 5 Kohlenstoffatomen, Alkoxyalkyl oder Alkylthioalkyl mit 2 bis 6 Kohlenstoffatomen oder Cycloalkyl mit 3 bis 6 Kohlenstoffatomen,
- $R^3$ und $R^4$ unabhängig voneinander Wasserstoff, Halogen, Nitro, Cyano, Alkyl, Alkoxy oder Alkylthio mit 1 bis 4 Kohlenstoffatomen und
- X Sauerstoff oder Schwefel bedeuten.

2. Trifluormethoxyphenyl-(di)thiophosphorsäureester der Formel I gemäß Anspruch 1, dadurch gekennzeichnet, daß $R^1$ Ethyl, $R^2$ Alkyl mit 3 oder 4 Kohlenstoffatomen, $R^3$ und $R^4$ unabhängig voneinander Wasserstoff oder Chlor und X Sauerstoff oder Schwefel bedeuten.
3. O-Ethyl-S-n-propyl-O-(4-trifluormethoxyphenyl)-thiophosphat.
4. O-Ethyl-S-n-propyl-O-(2-trifluormethoxy-phenyl)-thiophosphat.
5. O-Ethyl-S-n-propyl-O-(3-trifluormethoxyphenyl)-thiophosphat.
6. Schädlingsbekämpfungsmittel, enthaltend einen Trifluormethoxyphenyl-(di)thiophosphorsäureester der Formel I gemäß Anspruch 1.
7. Schädlingsbekämpfungsmittel, enthaltend inerte Zusatzstoffe und einen Trifluormethoxyphenyl-(di)thiophosphorsäureester der Formel I gemäß Anspruch 1.
8. Verfahren zur Bekämpfung von Schädlingen, dadurch gekennzeichnet, daß man eine wirksame Menge eines Trifluormethoxyphenyl-(di)thiophosphorsäureesters der Formel I gemäß Anspruch 1 auf die Schädlinge und/oder deren Lebensraum einwirken läßt
9. Verfahren zur Herstellung von Trifluormethoxyphenyl-(di)thiophosphorsäureestern der Formel I gemäß Anspruch 1, dadurch gekennzeichnet, daß man ein O,S-Dialkylphosphorsäureesterchlorid der Formel

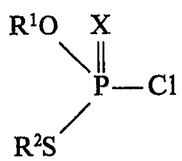

(II)

in der $R^1$, $R^2$ und X die im Anspruch 1, angegebenen Bedeutungen haben, gegebenenfalls in Gegenwart eines säurebindenden Mittels und in Gegenwart eines Verdünnungsmittels mit einem Trifluormethoxyphenol der Formel

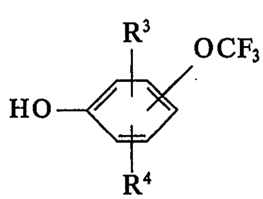

(III)

in der $R^3$ und $R^4$ die im Anspruch 1 genannten Bedeutungen haben, oder gegebenenfalls in Gegenwart eines Verdünnungsmittels mit einem Salz eines Trifluormethoxyphenols der Formel III umsetzt.

**Patentansprüche für den Vertragsstaat: AT**

1. Schädlingsbekämpfungsmittel, enthaltend inerte Zusatzstoffe und einen Trifluormethoxyphenyl-(di)thiophosphorsäureester der Formel

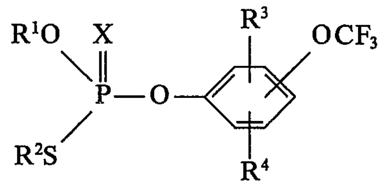

(I)

in der

- $R^1$ Alkyl mit 1 bis 3 Kohlenstoffatomen,
- $R^2$ Alkyl oder Halogenalkyl mit 1 bis 5 Kohlenstoffatomen, Alkoxyalkyl oder Alkylthioalkyl mit 2 bis 6 Kohlenstoffatomen oder Cycloalkyl mit 3 bis 6 Kohlenstoffatomen,
- $R^3$ und $R^4$ unabhängig voneinander Wasserstoff, Halogen, Nitro, Cyano, Alkyl, Alkoxy oder Alkylthio mit 1 bis 4 Kohlenstoffatomen und
- X Sauerstoff oder Schwefel bedeuten,

als Wirkstoff.

2. Schädlingsbekämpfungsmittel, enthaltend inerte Zusatzstoffe und einen Trifluormethoxyphenyl-(di)thiophosphorsäureester der Formel I gemäß Anspruch 1, dadurch gekennzeichnet, daß $R^1$ Ethyl, $R^2$ Alkyl mit 3 oder 4 Kohlenstoffatomen, $R^3$ und $R^4$ unabhängig voneinander Wasserstoff oder Chlor und X Sauerstoff oder Schwefel bedeuten, als Wirkstoff.

3. Schädlingsbekämpfungsmittel, enthaltend inerte Zusatzstoffe und O-Ethyl-S-n-Propyl-O-(4-trifluormethoxyphenyl)-thiophosphat als Wirkstoff.

4. Schädlingsbekämpfungsmittel, enthaltend inerte Zusatzstoffe und O-Ethyl-S-n-propyl-O-(2-trifluormethoxyphenyl)-thiophosphat.

5. Schädlingsbekämpfungsmittel, enthaltend inerte Zusatzstoffe und O-Ethyl-S-n-propyl-O-(3-trifluormethoxyphenyl)-thiophosphat.

6. Schädlingsbekämpfungsmittel nach Anspruch 1, dadurch gekennzeichnet, daß es 0,1 bis 95 Gew.-% Wirkstoff enthält.

7. Verfahren zur Herstellung von Trifluormethoxyphenyl-(di)thiophosphorsäureestern der Formel I gemäß Anspruch 1, dadurch gekennzeichnet, daß man ein O,S-Dialkylphosphorsäureesterchlorid der Formel

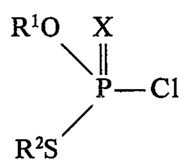

(II)

in der $R^1$, $R^2$ und X die im Anspruch 1 angegebenen Bedeutungen haben,
gegebenenfalls in Gegenwart eines säurebindenden Mittels und in Gegenwart eines Verdünnungsmittels mit einem Trifluormethoxyphenol der Formel

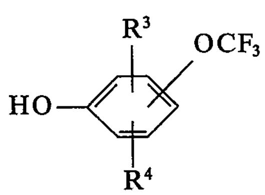

(III)

in der $R^3$ und $R^4$ die im Anspruch 1 genannten Bedeutungen haben,
oder gegebenenfalls in Gegenwart eines Verdünnungsmittels mit einem Salz eines Trifluormethoxyphenols der Formel III umsetzt.

**Claims for the contracting states: BE, CH, DE, FR, GB, IT, LI, LU, NL, SE**

1. A. trifluoromethoxyphenyl-(di)thiophosphoric acid ester of the formula

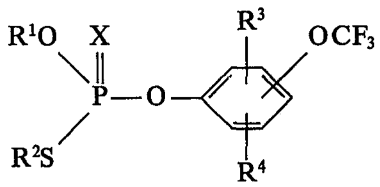

(I)

where

| | |
|---|---|
| $R^1$ | is alkyl of 1 to 3 carbon atoms, |
| $R^2$ | is alkyl or haloalkyl of 1 to 5 carbon atoms, alkoxyalkyl or alkylthioalkyl of 2 to 6 carbon atoms, or cycloalkyl of 3 to 6 carbon atoms, |
| $R^3$ and $R^4$ | independently of one another, are hydrogen, halogen, nitro, cyano or alkyl, alkoxy or alkylthio of 1 to 4 carbon atoms and |
| X | is oxygen or sulfur. |

2. A trifluoromethoxyphenyl-(di)thiophosphoric acid ester of the formula I as claimed in claim 1, wherein $R^1$ is ethyl, $R^2$ is alkyl of 3 or 4 carbon atoms, $R^3$ and $R^4$, independently of one another, are hydrogen or chlorine and X is oxygen or sulfur.

3. O-Ethyl-S-n-propyl-O-(4-trifluoromethoxyphenyl)-thiophosphate.

4. O-Ethyl-S-n-propyl-O-(2-trifluoromethoxyphenyl)-thiophosphate.

5. O-Ethyl-S-n-propyl-O-(3-trifluoromethoxyphenyl)-thiophosphate.

6. A pesticide containing a trifluoromethoxyphenyl-(di)thiophosphoric acid ester of the formula I as claimed in claim 1.

7. A pesticide containing inert additives and a trifluoromethoxyphenyl-(di)thiophosphoric acid ester of the formula I as claimed in claim 1.

8. A process for combating pests, wherein an effective amount of a trifluoromethoxyphenyl-(di)thiophosphoric acid ester of the formula I as claimed in claim 1 is allowed to act on the pests and/or their habitat.

9. process for the preparation of a trifluoromethoxyphenyl-(di)thiophosphoric acid ester of the formula I as claimed in claim 1, wherein an O,S-dialkyl thiophosphoric acid ester chloride of the formula

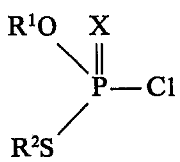

(II)

where $R^1$, $R^2$ and X have the meanings given in claim 1,
is reacted with a trifluormethoxyphenol of the formula

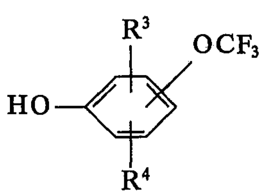

(III)

where $R^3$ and $R^4$ have the meanings given in claim 1,
in the presence or absence of an acid-binding agent and of a diluent, or with a salt of a trifluormethoxyphenol of the formula III in the presence or absence of a diluent.

**Claims for the contracting state: AT**

1. A pesticide containing inert additives and, as active ingredient, a trifluoromethoxyphenyl-(di)thiophosphoric acid ester of the formula

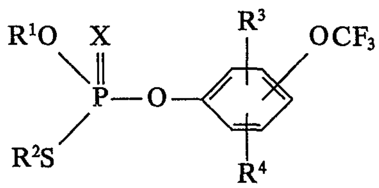

(I)

where

$R^1$ is alkyl of 1 to 3 carbon atoms,
$R^2$ is alkyl or haloalkyl of 1 to 5 carbon atoms, alkoxyalkyl or alkylthioalkyl of 2 to 6 carbon atoms, or cycloalkyl of 3 to 6 carbon atoms,
$R^3$ and $R^4$ independently of one another, are hydrogen, halogen, nitro, cyano or alkyl, alkoxy or alkylthio of 1 to 4 carbon atoms and
X is oxygen or sulfur.

2. A pesticide containing inert additives and, as active ingredient, a trifluoromethoxyphenyl-(di)thiophosphoric acid ester of the formula I as claimed in claim 1, wherein $R^1$ is ethyl, $R^2$ is alkyl of 3 or 4 carbon atoms, $R^3$ and $R^4$, independently of one another, are hydrogen or chlorine and X is oxygen or sulfur.

3. A pesticide containing inert additives and O-Ethyl-S-n-propyl-O-(4-trifluoromethoxyphenyl)-thiophosphate as active ingredient.

4. A pesticide containing inert additives and O-Ethyl-S-n-propyl-O-(2-trifluoromethoxyphenyl)-thiophosphate.

5. A pesticide containing inert additives and O-Ethyl-S-n-propyl-O-(2-trifluoromethoxyphenyl)-thiophosphate.

6. A pesticide as claimed in claim 1, containing from 0.1 to 95% by weight of active ingredient.

7. A process for the preparation of a trifluoromethoxyphenyl-(di)thiophosphoric acid ester of the formula I as defined in claim 1, wherein an O,S-dialkyl thiophosphoric acid ester chloride of the formula

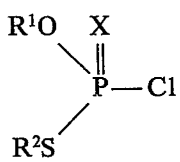

(II)

where $R^1$, $R^2$ and X have the meanings given in claim 1,
is reacted with a trifluoromethoxyphenol of the formula

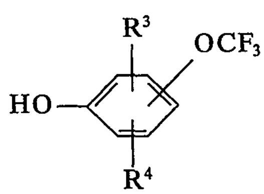

(III)

where $R^3$ and $R^4$ have the meanings given in claim 1,
in the presence or absence of an acid-binding agent and of a diluent, or with a salt of a trifluoromethoxyphenol of the formula III in the presence or absence of a diluent.

**Revendications pour les Etats contractants: BE, CH, DE, FR, GB, IT, LI, LU, NL, SE**

1. Esters d'acide trifluorométhoxyphényl-(di)thiophosphorique de la formule

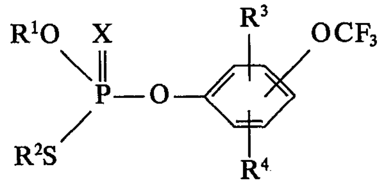

(I)

dans laquelle

$R^1$ désigne un radical alkyle en $C_1$ à $C_3$,
$R^2$ désigne un radical alkyle ou halo-alkyle en $C_1$ à $C_5$, alcoxy-alkyle ou alkyl-thio-alkyle en $C_2$ à $C_6$ ou cycloalkyle en $C_3$ à $C_6$,
$R^3$ et $R^4$ désignent chacun, indépendamment l'un de l'autre, un atome d'ydrogène ou d'halogène ou un groupe nitro, cyano, alkyle, alcoxy ou alkyl-thio en $C_1$ à $C_4$ et
X désigne un atome d'oxygène ou de soufre.

2. Esters d'acide trifluorométhoxyphényl-(di)thiophosphorique de la formule I selon la revendication 1, caractérisés en ce que $R^1$ = éthyle, $R^2$ = alkyle en $C_3$ ou $C_4$, $R^3$ et $R^4$, indépendamment l'un de l'autre, représentent H ou Cl et X = O ou S.
3. Le O-(4-trifluorométhoxyphényl)-thio-phosphate de O-éthyl-S-n-propyle.
4. Le O-(2-trifluorméthoxyphényl)-thio-phosphate de O-éthyl-S-n-propyle.
5. Le O-(3-trifluorométhoxyphényl)-thio-phosphate de O-éthyl-S-n-propyle.
6. Composition pesticide, contenant un ester d'acide trifluorométhoxyphényl-(di)thio-phosphorique de la formule I selon la revendication 1.
7. Composition pesticide, contenant des additifs inertes et un ester d'acide trifluorométhoxyphényl-(di)thio-phosphorique de la formule I selon la revendication 1.
8. Procédé de lutte contre les parasites, caractérisé en ce que l'on fait agir une quantité efficace d'un ester d'acide trifluorométhoxyphényl-(di)thiophosphorique de la formule I selon la revendication 1 sur les parasites et (ou) leur biotope.
9. Procédé de préparation d'esters d'acide trifluorométhoxyphényl-(di)thiophosphorique de la formule I selon la revendication 1, caractérisé en ce que l'on fait réagir le chlorure d'un O,S-dialkyl-phosphate de la formule

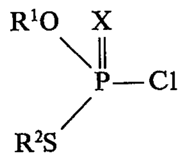

(II)

dans laquelle $R^1$, $R^2$ et X possèdent les significations définies dans la revendication 1,
en présence d'un diluant et éventuellement d'un fixateur d'acide, avec un trifluorométhoxy-phénol de la formule

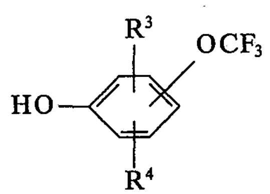

(III)

dans laquelle $R^3$ et $R^4$ possèdent les significations définies dans la revendication 1,
ou en présence d'un diluant éventuel avec un sel d'un trifluorométhoxy-phénol de la formule III.

**Revendications pour l'Etat contractant: AT**

1. Composition pesticide, contenant des additifs inertes et en tant que principe actif un ester d'acide trifluorométhoxyphényl-(di)thiophosphorique de la formule

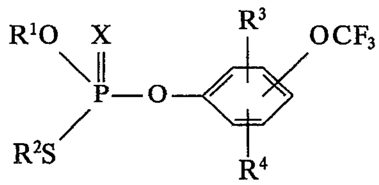

(I)

dans laquelle

$R^1$ désigne un radical alkyle en $C_1$ à $C_3$,
$R^2$ désigne un radical alkyle ou halo-alkyle en $C_1$ à $C_5$, alcoxy-alkyle ou alkyl-thio-alkyle en $C_2$ à $C_6$ ou cycloalkyle en $C_3$ à $C_6$,
$R^3$ et $R^4$ désignent chacun, indépendamment l'un de l'autre, un atome d'hydrogène ou d'halogène ou un groupe nitro, cyano, alkyle, alcoxy ou alkyl-thio en $C_1$ à $C_4$ et
X désigne un atome d'oxygène ou de soufre.

2. Composition pesticide, contenant des additifs inertes et en tant que principe actif un ester d'acide trifluorométhoxyphényl-(di)thiophosphorique de la formule I selon la revendication 1, caractérisé en ce que $R^1$ = éthyle, $R^2$ = alkyle en $C_3$ ou $C_4$, $R^3$ et $R^4$, indépendamment l'un de l'autre représentent H ou Cl et X = O ou S.

3. Composition pesticide, contenant des additifs inertes et en tant que principe actif le O-(4-trifluorométhoxyphényl)-thiophosphate de O-éthyl-S-n-propyle.

4. Composition pesticide, contenant des additifs inertes et en tant que principe actif le O-(2-trifluorométhoxyphényl)-thiophosphate de O-éthyl-S-n-propyle.

5. Composition pesticide, contenant des additifs inertes et en tant que principe actif du O-(3-trifluorométhoxyphényl)-thiophosphate de O-éthyl-S-n-propyle.

6. Composition pesticide suivant la revendication 1, caractérisée en ce ce qu'elle contient entre 0,1 et 95% en poids de principe actif.

7. Procédé de préparation d'esters d'acide trifluorométhoxyphényl-(di)thiophosphorique de la formule I selon la revendication 1, caractérisé en ce que l'on fait réagir le chlorure d'un O,S-dialkyl-phosphate de la formule

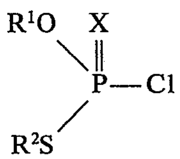

(II)

dans laquelle $R^1$, $R^2$ et X possèdent les significations définies dans la revendication 1,
en présence d'un fixateur d'acide, avec un trifluorméthoxy-phénol de la formule

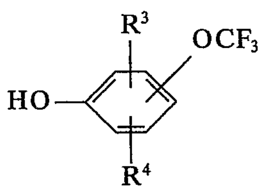

(III)

dans laquelle $R^3$ et $R^4$ possèdent les significations définies dans le revendication 1,
ou en préssence d'un diluant éventuel avec un sel d'un trifluorméthoxy-phénol de la formule III.